(19) Europäisches Patentamt
European Patent Office
Office européen des brevets

(11) Veröffentlichungsnummer: **0 505 818 A1**

# (12) EUROPÄISCHE PATENTANMELDUNG

(21) Anmeldenummer: **92104041.6**

(51) Int. Cl.[5]: **C07D 285/125**, //A01N43/82

(22) Anmeldetag: **10.03.92**

(30) Priorität: **23.03.91 DE 4109673**

(43) Veröffentlichungstag der Anmeldung: **30.09.92 Patentblatt 92/40**

(84) Benannte Vertragsstaaten: **BE CH DE ES FR GB IT LI NL**

(71) Anmelder: **BAYER AG**
**W-5090 Leverkusen 1 Bayerwerk(DE)**

(72) Erfinder: **Diehr, Hans-Joachim, Dr.**
**Höhe 35**
**W-5600 Wuppertal 11(DE)**
Erfinder: **Hammen, Günter, Dr.**
**Bunzlauer Weg 13**
**W-4049 Rommerskirchen(DE)**

## (54) Verfahren zur Herstellung von 2-Chlor-5-methylthio-1,3,4-thiadiazol.

(57) Das als Schädlingsbekämpfungsmittel oder als Zwischenprodukt zur Herstellung von Herbiziden verwendbare 2-Chlor-5-methylthio-1,3,4-thiadiazol kann in guten Ausbeuten und hoher Reinheit erhalten werde, indem man 2-Mercapto-5-methylthio-1,3,4-thiadiazol mit Phosgen ($COCl_2$) in Gegenwart eines Katalysators und gegebenenfalls in Gegenwart eines Verdünnungsmittels bei Temperaturen zwischen -20°C und +150°C umsetzt.



Rank Xerox (UK) Business Services

Die Erfindung betrifft ein neues Verfahren zur Herstellung von 2-Chlor-5-methylthio-1,3,4-thiadiazol, einer Verbindung, die als Schädlingsbekämpfungsmittel oder als Zwischenprodukt für Herbizide verwendet werden kann.

Es ist bekannt, daß man 2-Chlor-5-methylthio-1,3,4-thiadiazol erhält, wenn man 2-Amino-5-methylthio-1,3,4-thiadiazol mit Natriumnitrit in Salzsäure und in Gegenwart von Kupferbronze umsetzt (vgl. DE-OS 2144326). Als Nachteile für eine Anwendung dieser Herstellungsmethode im industriellen Maßstab sind die vielfach unbefriedigenden Produktausbeuten und die Notwendigkeit der Entsorgung größerer Chemikalienmengen, insbesondere von Salzsäure und Kupfer-Katalysator zu nennen.

Es wurde nun gefunden, daS man 2-Chlor-5-methylthio-1,3,4-thiadiazol der Formel (I)

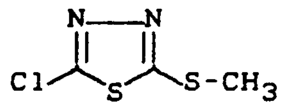

(I)

in guten Ausbeuten und in hoher Reinheit erhält, wenn man 2-Mercapto-5-methylthio-1,3,4-thiadiazol der Formel (II)

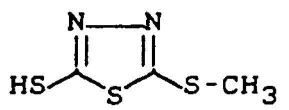

(II)

mit Phosgen ($COCl_2$) in Gegenwart eines Katalysators und gegebenenfalls in Gegenwart eines Verdünnungsmittels bei Temperaturen zwischen -20 °C und +150 °C umsetzt.

Überraschenderweise kann 2-Chlor-5-methylthio-1,3,4-thiadiazol der Formel (I) nach dem erfindungsgemäßen Verfahren in wesentlich besserer Ausbeute als nach der bekannten Methodik hergestellt werden, wobei die oben genannten Entsorgungsprobleme weitgehend vermieden werden können.

Der Reaktionsablauf beim erfindungsgemäßen Verfahren kann durch das folgende Formelschema skizziert werden:

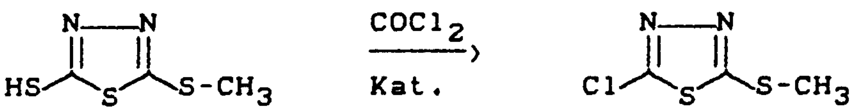


Die als Ausgangsstoff zu verwendende Verbindung der Formel (II) ist eine bekannte, handelsübliche Chemikalie.

Das erfindungsgemäße Verfahren wird in Gegenwart eines Katalysators durchgeführt. Geeignete Katalysatoren sind hierbei vor allem Stickstoffverbindungen, wie z.B. Triethylamin, N,N-Dimethyl-anilin, N,N-Dimethyl-benzylamin, Pyridin, 4-Dimethylamino-pyridin, N,N-Dimethyl-, N,N-Diethyl-, N,N-Dipropyl-, N,N-Diisopropyl -, N,N-Dibutyl- und N,N-Diisobutyl-formamid, N,N-Dimethyl-acetamid, N-Methyl-pyrrolidon und N-Formyl-piperidin, sowie Phosphorverbindungen, wie z.B. Tributylphosphin, Triphenylphosphin und Triphenylphosphinoxid.

Das erfindungsgemäße Verfahren wird vorzugsweise in Gegenwart eines Verdünnungsmittels durchgeführt. Als Verdünnungsmittel kommen dabei praktisch alle inerten organischen Solventien in Betracht. Hierzu gehören vorzugsweise aliphatische und aromatische, gegebenenfalls halogenierte Kohlenwasserstoffe, wie Benzol, Toluol, Xylol, Chlorbenzol, Dichlorbenzol, Methylenchlorid, Ethylenchlorid, Chloroform und Tetrachlormethan.

Die Reaktionstemperaturen können bei dem erfindungsgemäßen Verfahren in einem größeren Bereich variiert werden. Im allgemeinen arbeitet man bei Temperaturen zwischen -20 °C und +150 °C, vorzugsweise bei Temperaturen zwischen 0 °C und 120 °C.

Das erfindungsgemäße Verfahren wird im allgemeinen unter Normaldruck durchgeführt. Es ist jedoch

auch möglich, unter erhöhtem oder vermindertem Druck - im allgemeinen zwischen 100 mbar und 10 bar - zu arbeiten.

Zur Durchführung des erfindungsgemäßen Verfahrens setzt man auf 1 Mol 2-Mercapto-5-methylthio-1,3,4-thiadiazol im allgemeinen zwischen 1 und 5 Mol, vorzugsweise zwischen 1 und 3 Mol, Phosgen und zwischen 0,005 und 0,5 Mol, vorzugsweise zwischen 0,01 und 0,1 Mol, eines Katalysators ein.

Die Reaktionskomponenten können in beliebiger Reihenfolge vermischt werden.

In einer bevorzugten Ausführungsform des erfindungsgemäßen Verfahrens werden das 2-Mercapto-5-methylthio-1,3,4-thiadiazol, das Lösungsmittel und der Katalysator verrührt, Dann wird unter Rühren Phosgen eingeleitet und dabei die Mischung allmählich auf die erforderliche Reaktionstemperatur gebracht. Wenn praktisch kein Phosgen mehr aufgenommen wird, wird an Stelle von Phosgen Stickstoff durch die Mischung geleitet, um überschüssiges Phosgen zu verdrängen. Dann wird das Verdünnungsmittel unter vermindertem Druck abdestilliert und das als Rückstand verbleibende Produkt durch Vakuumdestillation gereinigt.

Das nach dem erfindungsgemäßen Verfahren herzustellende 2-Chlor-5-methylthio-1,3,4-thiadiazol kann als Schädlingsbekämpfungsmittel verwendet werden (vgl. DE-OS 2144326).

Nach Oxidation zu 2-Chlor-5-methylsulfinyl-1,3,4-thiadiazol oder 2-Chlor-5-methylsulfonyl-1,3,4-thiadiazol (vgl. die Herstellungsbeispiele) ist auch eine Verwendung zur Herstellung von Herbiziden möglich (vgl. EP-A 192117).

Herstellungsbeispiele:

Beispiel 1

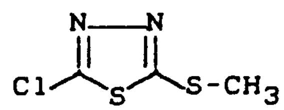

In eine auf 10° C abgekühlte Mischung aus 115 g (0,7 Mol) 2-Mercapto-5-methylthio-1,3,4-thiadiazol, 7 ml N-Formylpiperidin und 700 ml Chlorbenzol wird unter Rühren Phosgen eingeleitet und die Mischung wird nach ca. 30 Minuten innerhalb von ca. 2 Stunden allmählich auf 110° C aufgeheizt. Bei dieser Temperatur wird das Einleiten von Phosgen noch ca. 2 Stunden fortgesetzt. Nach Entfernen der Heizquelle wird statt Phosgen Stickstoff durch die Mischung geleitet und anschließend das Chlorbenzol im Vakuum weitgehend abdestilliert. Der Rückstand wird im Ölpumpenvakuum destilliert.

Man erhält 81,5 g (70% der Theorie) 2-Chlor-5-methylthio-1,3,4-thiadiazol vom Siedepunkt 78° C-79° C bei 0,5 mbar.

Folgeprodukte

Beispiel 2

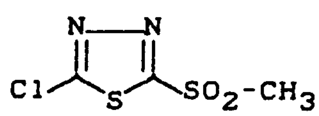

16,7 g (0,1 Mol) 2-Chlor-5-methylthio-1,3,4-thiadiazol werden in 100 ml Essigsäure gelöst und nach Zugabe einer Spatelspitze Natriumwolframat bei 25° C bis 35° C unter Außenkühlung tropfenweise mit 28,3 g (0,29 Mol $H_2O_2$) einer 35%igen wäßrigen Hydrogenperoxidlösung versetzt. Das Reaktionsgemisch wird 20 Stunden bei ca. 25° C gerührt, dann mit Wasser auf etwa das doppelte Volumen verdünnt; das hierbei kristallin angefallene Produkt wird durch Absaugen isoliert.

Man erhält 15,3 g (74% der Theorie) 2-Chlor-5-methylsulfonyl-1,3,4-thiadiazol vom Schmelzpunkt 58° C.

Beispiel 3

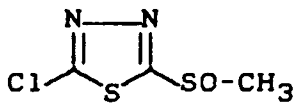

16,7 g (0,1 Mol) 2-Chlor-5-methylthio-1,3,4-thiadiazol werden in 25 ml Chloroform gelöst und nach Abkühlen auf -60 °C unter Rühren tropfenweise mit einer Lösung von 27 g (0,11 Mol) 3-Chlor-perbenzoesäure (70%ig) in 135 ml Chloroform versetzt. Das Reaktionsgemisch wird 20 Stunden bei ca. 20 °C gerührt und dann filtriert. Das Filtrat wird mit gesättigter wäßriger Natriumbicarbonat-Lösung gewaschen und dann wird im Wasserstrahlvakuum das Lösungsmittel sorgfältig abdestilliert.

Man erhält 13 g (61% der Theorie) 2-Chlor-5-methylsulfinyl-1,3,4-thiadiazol als öligen Rückstand (Gehalt: 84%).

## Patentansprüche

1. Verfahren zur Herstellung von 2-Chlor-5-methylthio-1,3,4,-thiadiazol der Formel (I),

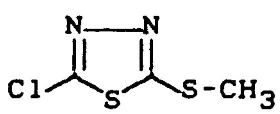

(I)

dadurch gekennzeichnet, daß man 2-Mercapto-5-methylthio-1,3,4-thiadiazol der Formel (II)

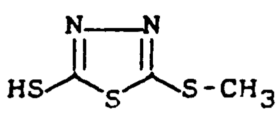

(II)

mit Phosgen ($COCl_2$) in Gegenwart eines Katalysators und gegebenenfalls in Gegenwart eines Verdünnungsmittels bei Temperaturen zwischen -20 °C und +150 °C umsetzt.

2. Verfahren nach Anspruch 1, dadurch gekennzeichnet, daß man bei Temperaturen zwischen 0 °C und +120 °C arbeitet.

3. Verfahren nach Anspruch 1, dadurch gekennzeichnet, daß man auf 1 Mol 2-Mercapto-5-methylthio-1,3,4-thiadiazol (II) 1 bis 5 Mol Phosgen und 0,005 bis 0,5 Mol eines Katalysators, vorzugsweise 1 bis 3 Mol Phosgen und 0,01 bis 0,1 Mol eines Katalysators einsetzt.

4. Verfahren nach Anspruch 1, dadurch gekennzeichnet, daß man als Katalysator eine Stickstoffverbindung, vorzugsweise ausgewählt aus der folgenden Gruppe:

   Triethylamin, N,N-Dimethyl-anilin, N,N-Dimethylbenzylamin, Pyridin, 4-Dimethylamino-pyridin, N,N-Dimethyl-, N,N-Diethyl-, N,N-Dipropyl-, N,N-Diisopropyl -, N,N-Dibutyl-und N,N-Diisobutyl-formamid, N,N-Dimethyl-acetamid, N-Methyl-pyrrolidon und N-Formyl-piperidin,

   oder eine Phosphorverbindung, vorzugsweise ausgewählt aus der folgenden Gruppe:

   Tributylphosphin, Triphenylphosphin und Triphenylphosphinoxid,

   einsetzt.

5. Verfahren nach Anspruch 1, dadurch gekennzeichnet, daß man in Gegenwart eines Verdünnungsmittels arbeitet.

6. Verfahren nach Anspruch 5, dadurch gekennzeichnet, daß man als Verdünnungsmittel ein inertes organisches Lösungsmittel, vorzugsweise ausgewählt aus der Gruppe der aliphatischen und aromatischen, gegebenenfalls halogenierten Kohlenwasserstoffe, verwendet.

7. Verfahren nach Anspruch 6, dadurch gekennzeichnet, daß man als inertes organisches Lösungsmittel Benzol, Toluol, Xylol, Chlorbenzol, Dichlorbenzol, Methylenchlorid, Ethylenchlorid, Chloroform oder Tetrachlormethan verwendet.

Europäisches Patentamt

# EUROPÄISCHER RECHERCHENBERICHT

Nummer der Anmeldung

EP 92 10 4041

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | DE-A-2 144 326 (AGRIPAT S.A.)<br>* das ganze Dokument *<br>--- | 1-7 | C07D285/125<br>//A01N43/82 |
| X | GB-A-913 910 (BADISCHE ANILIN-& SODA-FABRIK AKTIENGESELLSCHAFT)<br>* das ganze Dokument * | 1-7 | |
| Y | * das ganze Dokument *<br>----- | 1-7 | |
| | | | RECHERCHIERTE SACHGEBIETE (Int. Cl.5) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 26 JUNI 1992 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)